# EUROPEAN PATENT APPLICATION

(11) **EP 1 529 507 A1**
(43) Date of publication of application: **11.05.2005**
(21) Application number: 03025595.4
(22) Date of filing: 07.11.2003
(51) Int. Cl.: A61F 13/15

(54) **An ergonomically packaged absorbent article**

(71) Applicant: THE PROCTER & GAMBLE COMPANY, Cincinnati, Ohio 45202 (US)
(72) Inventor: Engel, Roland, 65843 Sulzbach (DE); Schwinn, Walter, 53894 Mechernich (DE); Simon, Beate, 65760 Eschborn (DE); Stelzig, Lutz, 60489 Frankfurt am Main (DE); Zimmer, Dirk, 63879 Euskirchen (DE)
(74) Representative: Hirsch, Uwe Thomas M.H.

(57) **Abstract**

The present invention relates to packaged absorbent articles, which are disposable absorbent hygiene articles especially as incontinence care articles like baby-, adolescent- or adult- diapers or training pants, diapering inserts. Such articles are conventionally packaged in bulk quantities such as a household quantity (for example a weekly of fortnightly average consumption quantity of articles) or in institutional quantities of several time the household quantity. The present invention is based on the recognition that there is a need for individually packaged or very small quantity packaged articles. The improvement provided by the present invention is based on the recognition that the article configuration of bulk packaged articles is unsuitable for individually packaged or very small quantity packaged articles. It has been found that basic article performance, article usage, package material consumption, manufacturing efficiency and energy consumption, marketability of the article, economic aspects of providing articles to end-users, individually or in combinations of these aspects can be improved when providing the articles in accordance with the present invention for substantially individually packaged articles.

## Description

### Field of the invention

The present invention relates to packaged absorbent articles, which are disposable absorbent hygiene articles such as incontinence care articles like baby-, adolescent- or adult- diapers or training pants, diapering inserts, adult incontinence devices, or feminine care articles like sanitary napkins, panty liners, or other absorbent hygiene articles like sweat pads for shirts or blouses, collars for hats and the like. Such articles are conventionally packaged in bulk quantities such as a household quantity (for example a weekly of fortnightly average consumption quantity of articles) or in institutional quantities of several time the household quantity. The present invention is based on the recognition that there is a need for individually packaged or very small quantity packaged articles. The improvement provided by the present invention is based on the recognition that the article configuration of bulk packaged articles is unsuitable for individually packaged or very small quantity packaged articles. It has been found that basic article performance, article usage, package material consumption, manufacturing efficiency and energy consumption, marketability of the article, economic aspects of providing articles to end-users, individually or in combinations of these aspects can be improved when providing the articles in accordance with the present invention for individually packaged or very small quantity packaged articles.

### Background of the invention

Disposable absorbent hygiene articles as defined above are well known in the art, and significant effort has been spent against improving their performance. Such improvements, particularly for articles like baby-, adolescent- or adult- diapers or training pants and diapering inserts, generally aim at addressing the primary function of such articles, namely retaining body fluids. It is however also important to provide such articles in a fashion in which the user of such articles does not have to overcome barriers for the usage based in his/her individual situation. For example in the case of a baby diaper or training pant it is important to have the article available at the time of need such as away-from-home, and to have the article available in hygienic form. This can be achieved by individual packaging of the article.

Similarly in regions where commercialization of articles is only meaningful or possible in small quantities, e.g. in away-from-home situation such as highway rest stations or in regions where buyers of such articles cannot afford to buy the articles in bulk quantities, individually packaging of the article would resolve the needs of consumers. Also storage and transport aspects of individually packaged articles are much better than for bulk-packaged articles, e.g. it is more convenient to have only a small quantity of individual packaged diapers on a baby changing table than having to find the space for a bulk-packaged weekly quantity, while simultaneously improving hygiene of the article. Individually packaged articles also allow significant improved flexibility for the manufacturer and distributor of such articles in selecting and changing sales-package quantities and optimizing storage and transport capacities.

Despite all these benefits individual packaging of articles has not found commercial success and has not been selected by manufacturers, apparently on the basis that such packaging would not be attractive enough to consumers to justify associated changes in packaging. However there were numerous attempts to create individual packaging.

For example in US 5,071,414 a packaging pocket in the form of a rolled diaper configuration for disposal is disclosed. The user of the diaper creates this condition after use for convenient and hygienic disposal of the used article. Similar ideas have previously been disclosed in US 3,963,029, EP 893 115, and EP 374 730 all intending to provide a rolled diaper configuration for disposal after usage.

Individual packaging of fresh articles, especially for sanitary napkins is disclosed e.g. in JP 09154878, entitled 'Packaging structure for absorptive article', EP 699427, entitled 'Individually rolled sanitary towel, JP 2001 087 306, entitled 'Individual package of absorbent article', WO 99 52484 entitled 'Individually wrapped sanitary napkin', or WO 93 21878 entitled 'Individually packaged sanitary napkin having cleansing wipe packaged therewith'.

In EP 680304, entitled 'Absorbent product provided in roll form' a large quantity of articles, which are attached to each other by perforations, is disclosed. Finally in US 4,770,298 entitled 'Packaging of absorbent products' a dispenser for absorbent articles, which can be rolled around a core and are connected to each other, is disclosed (similar to toilet or bathroom tissues).

In another aspect of the present invention it is noted that especially in the field of baby diapers or training pants the development of thinner and more comfortable articles, due to new and less material but also due to better distribution of such materials, has been considered beneficial for packaging aspects, making such a development the subject of substantial commercial interest. For example, thinner diapers are not just less bulky to wear and fit better under clothing of a wearer, they should also be more compact in a bulk package, making it easier for the consumer to carry and store. Compactness in packaging also results in reduced distribution costs for the manufacturer and distributor, including less shelf space required in the store per article unit. These benefits were originally achieved by compressing the diapers in their bulk configuration to squeeze out air (see e.g. EP 780 325), which was possible due to the large quantities of e.g. foams or pulp fiber structures in the design of the compressed articles without causing hardspots or other detriment to the articles.

However this benefit has been limited somewhat by the more recent material and design developments on absorbent articles as mentioned above because of the usage of more of the new non-compressible materials replacing compressible materials. More importantly thinness was also achieved by designing absorbent structures to have a material distribution of differing quantities along the length and width of the article. This achieves thinness of the articles during use, but makes compression in bulk-packages, where the article is usually either flat packaged or folded once along its length, and then compressed more difficult, because the material quantity for compression changes across the compressed surface. This either results in less compression than possible on average being achieved, or if compression was set equivalent the overall material reduction, in article quality defects by creating hard spots in some regions of the article due to the high compression.

To address this it could be suggested to fold absorbent articles more than once along its length and thereby create an averaging of the material quantities along the article length (often referred to as tri-folding or even quad-folding). However each fold in an article has potentially adverse effects in liquid transport (creating creases in the main liquid transport path). Again this could be addressed by appropriate unfolding techniques, but without control, how the article is unfolded, a certain variance in article performance is left to chance. Also consumers have found the handling of tri- or quad-folded articles unwieldy and cumbersome.

Accordingly, it would be desirable to be able to provide an absorbent article addressing some or all of the above mentioned concerns and providing an alternative to current conventional bulk packaging of absorbent articles, particularly diapers and training pants. Primarily it is important to provide packaged absorbent articles in a fashion, which is attractive to the consumer and minimizes product performance variations. Also it is important that due to the low attention assigned by consumers of absorbent articles to the packaging of such articles any packaging improvement is economically balanced versus the benefits it achieves and minimizes or prevents introduction of handling and/or use negatives for manufacturers or distributors or consumers or users of the article.

Hence it is an object of the present invention to provide substantially individually packaged absorbent articles, such that the package provides a handling and/or use improvement, especially in respect to package volume and package handling.

### Summary of the invention

The present invention and its characteristics are fully defined in the independent claims and preferred embodiments are specified in the dependant claims and in the detailed disclosure of this specification.

In particular the present invention relates to packaged absorbent articles, which are disposable absorbent hygiene articles such as incontinence care articles like baby-, adolescent- or adult- diapers or training pants, diapering inserts, adult incontinence devices, or feminine care articles like sanitary napkins, panty liners, or other absorbent hygiene articles like sweat pads for shirts or blouses, collars for hats and the like. Preferred are incontinence care articles, especially baby-, adolescent- or adult-diapers or training pants.

The packaged article is provided in an ergonomically shaped package. This package is substantially of cylindrical shape, having a length of between 38 mm and 580 mm and an equivalent circumferential diameter (ECD), which is between 22.8 mm and 85.4 mm. Preferably the length of the packaged article is at least 52 mm, preferably at least 85 mm and most preferably at least 127 mm. The length is preferably up to 420 mm, preferably up to 310 mm and most preferably up to 210 mm.

Preferably the ECD is at least 28.5 mm, more preferably 34.2 mm, and most preferably at least 38 mm. preferably the ECD is no more than 76.25 mm, more preferably no more than 68.3 mm, and most preferably no more than 63 mm.

In a preferred embodiment of the packaged absorbent article according to the present invention the cylindrical shape is created by a smallest and by a largest actual diameter along the length of the article. It is preferred, that the difference between the smallest and the largest diameter at any point of length of the packaged article is less than 25 %, more preferably less than 15 % and most preferably less than 5% of the smallest diameter.

In a further preferred embodiment according to the present invention the substantially cylindrical shape of the packaged absorbent article is made such, that the diameter changes ergonomically along the length of the cylindrical form to accommodate the grip pattern of four fingers opposite one thumb of a hand, similar to those shapes found in control grips of computerized navigation systems or computer game consoles (joy sticks). It is mostly preferred, that the grip pattern of four fingers and one thumb is that of a left hand.

It is a further preferred embodiment according to the present invention, that the packaged absorbent article is a single article, possibly in conjunction with an additional item, selected from the group of information or advertisement leaflets, disposal bags, change mats, dry or wet wipes, toys or gimmicks or combinations thereof. In a particular embodiment, especially useful for marketing promotions, according to the present invention the packaged absorbent article is packed with an additional item, which is another absorbent article, preferably two identical, disposable absorbent articles such as diapers are provided, such that the quantity of diapers of a single package allows use of the articles for twins.

The packaged absorbent article according to the present invention preferably is rolled up into the generally cylindrical form. In one embodiment the article may be folded along one, two or three axes parallel to the length of the article prior to being rolled-up. If the article is a diaper or training pant and the article is rolled it is preferred to first fold the article along an axis perpendicular to the article's length, roll the article from the fold towards the ends of the article such that the ends of the article in the folded configuration are overlaying each other in a coextensive fashion.

In a preferred embodiment the article in its cylindrical form can be wrapped into a film or paper package material to maintain the article in the cylindrical form and protect the article from the environment. Most preferably the package material is a shrink film material. It is also preferred, that when the article is a diaper or a diaper pant, the packaged and rolled-up absorbent article does not have a pressure difference in the packaged article of more than 25 %, preferably not more than 15 % and most preferably not more than 10 % between any two points along the length of the packaged article, based on the lower of any two pressures measured.

Further embodiments and their beneficial aspects will be apparent from the following detailed description of the invention, in which a packaged absorbent diaper is used as an example for exemplifying and disclosing the details of the present invention.

### Detailed description of the invention

### A. Definitions

As used herein, the term "packaged absorbent article" refers to an absorbent article, which is in a configuration intended to be maintained for shipment, transport and sales of the article to the final user of the article by a package component.

As used herein, the term "cylinder" refers to a shape or form of generally cylindrical shape, without mathematically exact reflection of a cylinder. Therefore as used herein e.g. the shape of a cone, a stump of a cone, or a column (straight or slightly bent), based e.g. on a triangle or multi-angle shape with rounded edges, would qualify as cylindrical. A cylinder has two end surfaces, which have center points. In case of asymmetrical surfaces the gravimetrical center points are used as the center points. A cylinder also has a centerline, which is the line connecting the center points of both cylinder end surfaces with one another.

As used herein, the term "actual diameter (of a cylinder)" refers to the length of a line between two walls of a cylinder, which line crosses perpendicular through the centerline of the cylinder. For ideal cylinders all diameters are equal, for actual cylinders according to the present invention, a largest and smallest diameter can be defined at any point of the cylinder centerline.

As used herein, the term "equivalent circumferential diameter of a cylinder" refers to the diameter of a circle perpendicular to the centerline of a cylinder at a point of the center line, which circle has the same circumference as the actual cylinder at the same point along its centerline.

As used herein, the term "equivalent circumferential diameter" also referred to as "ECD" is used in the context of a packaged absorbent article and refers to the largest equivalent circumferential diameter of the cylinder from of said packaged absorbent article.

As used herein, the term "length of a cylinder" is the length of the centerline of a cylinder.

As used herein, the terms "region(s)" or "zone(s)" of an element refer to portions or sections of that element.

As used herein, the term "comprising" means that e.g. various components, members, steps, and the like can be conjointly employed according to the present invention. Accordingly, the term "comprising" encompasses the more restrictive terms "made of" and "consisting of," these latter, more restrictive terms having their standard meaning as understood in the art.

All percentages, ratios, and proportions used herein are by weight unless otherwise specified.

### B. The Absorbent Article

The specific design of the absorbent article according to the present invention is not in all instances dominating the present invention. Hence any generally well-known absorbent article of the prior art can be the subject of the present invention. According to the present invention especially diapers and training pants (also referred to interchangeably as diaper pants) are articles, which benefit from the technology of the present invention and are therefore described as examples for absorbent articles.

Disposable absorbent diapers and training pants are articles for absorption of urine (also referred to as disposable absorbent incontinence articles). These absorbent articles typically comprise a liquid impervious (but preferably gas pervious) backsheet, a fluid pervious topsheet, and an absorbent core between the backsheet and the topsheet. The topsheet is usually joined to, or otherwise associated with the backsheet along the periphery of the two sheets extending beyond the absorbent core. Such articles are well known in the art and fully disclosed in various documents mentioned throughout the description e.g. in EP 752 892. Especially when the articles are designed for optimum material usage and thereby thinness during use can be provided in a way such that the core and the article are not of constant thickness.

The absorbent article in a flat, stretched out condition is substantially rectangular. It can have portions cut out of the general rectangular shape to enhance fit for the specific usage conditions intended (such as side notches in diapers for better leg fit). In general the rectangular shape has a length and a width (in a diaper/training pant context the length of the diaper is in the direction, which in use reaches from the front to the rear of the wearer, while the width is perpendicular thereto).

The difference between diapers and training pants is that diapers are folded around a wearer and closed by a closure system usually at the sides of the article, while training pants (in accordance with the word pants) are already in a closed condition and are pulled up onto the wearer. The closure of training pants can be achieved by adhesive means, by welding, seaming (ultrasonic, pressure or otherwise) or by mechanical means or combinations thereof. Sometimes it is preferable if there is a permanent, but breakable closure of the pant together with a re-closable attachment means, allowing convenient opening and re-closure of a training pant. The closure system of diapers conventionally comprises a re-closable adhesive or mechanical closure system or combination thereof It should be noted that one of the limiting aspects in exerting pressure to conventional diapers during packaging is the destructive effect on mechanical hook-loop fastener systems (whether used in diapers or training pants). Given the improvements of the present invention in respect to distribution of pressures within the packaged article such limitations are at least reduced if not eliminated. Hence articles comprising mechanical fasteners of the hook loop type are particularly susceptible to the present invention and are therefore preferred.

The absorbent article conventionally can be folded along 2 axes parallel to the length of the article to reduce its width for packaging. In the context of diapers/training pants the resulting width will define the length of the cylindrical shape of the packaged absorbent article. The article in a preferred embodiment is then folded along an axis perpendicular to the length of the article. For rolling the article from this fold it is preferred that the fold is placed such that after rolling the article to a cylindrical shape the 2 ends of the article overlap each other either exactly coextensively or such that the outer end extends beyond the inner end when the article is rolled up. This provides a hygienic benefit and allows e.g. graphical symbols on the outside of the outer end to appear quite clearly and unobstructed on the outer surface of the cylindrical shaped packaged article. Of course this last benefit is only of relevance if the packaging material used (in accordance with the present invention) allows visible inspection of graphical symbols on the outside of the rolled up article. Rolling the article from the center also prevents that a too minute center rolling is created and causes article performance drawbacks such as rolling end flaps in the case of a diaper or sticking of a panty fastening adhesive to skin in the case of a sanitary napkin.

When being rolled up the article can be loosely rolled or preferably it can be rolled to create a compression of the article to create a denser, more compact packaging. The benefit of rolling is of course that any thickness differences of the article in its length direction will be negligible because the resulting pressure is equalized across the radius. In other words e.g. a baby diaper which is thicker in the center than at the ends, can be compressed with a force in radial direction when packaged according to the present invention, this force will cause a compression substantially equal at the tick and at the less thick regions of the article along its length. Compression differences due to thickness differences across the width of the article (which relates to the length of the packaged article) are usually much smaller and can therefore be easier accepted without detrimental hardspots or other drawbacks. In particular by appropriate selection of longitudinal folds, especially when the article is a diaper or a training pant, the skilled person can ensure that the packaged and rolled-up absorbent article does not have a pressure difference in the packaged article of more than 25 %, preferably not more than 15 % and most preferably not more than 10 % between any two points along the length of the packaged article, based on the lower of the two pressures measured. (the pressure can be measured by evaluation of the force contained by the circumscribing package material, e.g. by use of a conventional tensile or pressure measurement instrument).

### C. The Package

The package can be made of any material suitable in the context of absorbent articles. It primarily needs to satisfy the function of providing stability to the cylindrically shaped article. If the article is rolled a simple tape would satisfy the requirement but for hygiene reasons would not be most preferred (same for a string or rubber band around the rolled article).

Preferred are package materials capable of completely enclosing the article in its cylindrical shape such as paper or polymeric films. Preferred are especially paper sleeves for packages where the compression for the cylindrical shape of the article is very high as paper has an excellent stress strain behavior. On the other hand similar behavior for articles slightly less compressed (i.e. without reaching the limit where product performance is substantially endangered) can be found in polymeric film materials, where the stress strain curve for the relevant pressures is relatively steep, approximately linear and without significant time dependence or temperature dependence (time in this context should be considered the average time between packaging of the article and use of the article - this can be estimated for diapers/training pants to be up to 3 month; temperature in this context should be the usual temperatures which the packaged article is exposed to prior to usage - this can be as high as e.g. 50 °C in a car exposed to direct sunshine).

The key benefit of polymeric film packages is that they can easily be sealed around the cylinderically shaped article even when under compression. Also such films are available in transparent form such that visual inspection of the content is possible without the need for a opening in the package. This would also allow including usage instruction leaflets or other information notes on the outside of the cylindrically shaped article but inside the package. A highly preferred film packaging material is a shrink wrap, which can be provided as a sleeve around the article, can be sealed at both ends of the cylinder and can then be exposed to enough heat to shrink exactly to the shape and dimensions of the cylindrical article.

According to the present invention only individually packaged articles are claimed. However inclusion of additional other items is within the invention. Such an additional item can be the above-mentioned leaflet or other useful tools for usage with the article or for marketing purposes as disclosed herein. There is one exception to the individual packaging according to the invention, namely when the additional item is another article similar or preferably identical to the first one, so as to provide a simultaneous double usage option, especially in the context of diapers for twins.

For shipment of large quantities of packaged articles according to the present invention it will be necessary to provide them in an outer package, such as a carton or a bag, or as a bundle. It is a clear benefit of the present invention that contrary to previous bulk packaging the dimensions of such outer package or bundle only depends on the length of the cylinder forms of the packaged absorbent article (which directly relates to the width of the article). In previous bulk packaging both the width and length of the article were decisive on the outer package dimensions. Also with individually packaged articles sale of such articles by the piece, usage of dispensing machines (e.g. coin operated or wall mounted in an at home situation at the change table), or in display dispensers in stores become an option.

### D. The Shape of the Packaged Absorbent Article

A key aspect in the present invention is the recognition that most of the compact shapes known from the prior art were used for disposal of the article or if they were used for fresh articles they were known for bulk packages of articles (such as rolls of interconnected articles). In this context it is not surprising that no attention has been given to the handling aspects of substantially individually packaged articles. Data are available as shown in the following Table 1 giving the dimensions of cylinders which can just be circumscribed by women's hands between thumb and middle finger or between thumb and index finger.

**Table 1**

| Thumb to middle finger grip diameter | | |
|---|---|---|
| Country, sex, age (years) | Size of 1^{st} %ile | Size of 99^{th} %ile |
| UK, female, 18-39 | 38 mm | 53 mm |
| German, female, 18-64 | 39 mm | 53 mm |
| USA, female, 18-39 | 38 mm | 53 mm |
| Japan, female, 18-55 | 38 mm | 46 mm |

The values describe the diameter of a circular cylinder, which the women, whose data were taken, were able to grip when thumb and middle finger just touched. 1^{st} %ile means only 1% of participants had a grip diameter value of the indicated size or smaller. 99^{th} %ile means 99% of participants had a grip diameter value of the indicated size or smaller. This table is based on data obtained from the inter-net according to "People Size 2000", which is a database of human sizes. On the date of filing it is available under the inter-net address: http://www.openerg.com/psz.htm.

Data for thumb to index finger grip diameter are also available. They are approximately 8mm smaller than the thumb to middle finger grip diameter values.

From these data and evaluations by the inventors of the present application it has been found that the dimensions as defined herein for ECD and length provide packages, which are preferred over conventionally packaged or alternative individually absorbent articles. The length and diameter of the cylindrical shaped package have to be selected to allow and facilitate an improved handling. This has to be satisfied in situations ranging from picking the article up from a horizontal surface, taking the article out of a roll dispenser, or out of a confined space (such as a carton or handbag), or when taking it from another person's hand.

In this context the ratio between length and diameter has also been found to be of relevance in some cases, especially when coming to very small diameters. It has been found that length to diameter ratios approaching the so called 'golden section' of 2/3 to 1/3 are most preferred. Hence in preferred embodiments ratios of length to diameter should be between 6:1 to 1:1, more preferably between 4.5:1 to 1.25:1, most preferably between 3:1 to 1.5:1. Care should be taken that the length continues to allow easy holding of the cylinder and passing it on to another hand. These ratios may numerically not be found exactly reflected by the values found by the inventors for the length and ECD. This can be attributed due to the evaluation of shapes, which were not ideal cylinders but included shapes with some actual diameter variation. Hence the length to diameter ratios are given for ideal cylinders but can in a first approximation be transferred to generally cylindrical shapes according to the present invention, as long as the diameter and length values remain inside the values as defined herein.

In a preferred embodiment of the packaged absorbent article according to the present invention the cylindrical shape is created by a smallest and by a largest actual diameter along the length of the article. In preferred embodiments the difference between the smallest and the largest diameter at any point of length of the packaged article should be less than 25 % or as otherwise defined herein. It is of course particularly preferred to provide the shape of the packaged article in a well known ergonomically shaped design. In this context it is considered to be desirable to provide the substantially cylindrical shape of the packaged absorbent article such, that the diameter changes ergonomically along the length of the cylindrical shape to accommodate the grip pattern of four fingers opposite one thumb of a hand, similar to those shapes found in control grips e.g. of navigation systems or computer game consoles (joy sticks). If so it is considered most preferred that the grip pattern is that of a left hand.

## Claims

1. A packaged absorbent article, preferably said article being a disposable absorbent hygiene article, more preferably said article being a diaper or a training pant, said packaged article being provided in an ergonomic form, said form being of substantially cylindrical shape, having a length and having an equivalent circumferential diameter (ECD), both as defined herein, said packaged article being **characterized in that** said length is between 38 mm and 580 mm and said ECD is between 22.8 mm and 85.4 mm.

2. A packaged absorbent article according to claim 1 **characterized in that** said length is at least 52 mm, preferably at least 85 mm and most preferably at least 127 mm, and further **in that** said length is up to 420 mm, preferably up to 310 mm and most preferably up to 210mm.

3. A packaged absorbent article according to any preceding claims **characterized in that** said ECD is at least 28.5 mm, preferably at least 34.2 mm and most preferably at least 38 mm and **in that** said ECD is no more than 76.25 mm, preferably no more than 68.3 mm and most preferably no more than 63 mm.

4. A packaged absorbent article according to any preceding claims **characterized in that** said form at any point along said length has a smallest and a largest actual diameter and **in that** the difference between smallest and largest diameter at any point along said length is less than 25 %, preferably less than 15%, most preferably less than 5% of the largest diameter.

5. A packaged absorbent article according to any preceding claims **characterized in that** said packaged article is provided in a form which changes ergonomically along said length to accommodate the grip pattern of four fingers opposite one thumb of a hand, preferably of a left hand.

6. A packaged absorbent article according to any preceding claims **characterized in that** said article is rolled up into said form and packaged by a film material, preferably by a shrink film material.

7. A packaged absorbent article according to any preceding claims **characterized in that** said article is folded along one, two, or three axes parallel to said length prior to being packaged.

8. A packaged absorbent article according to any preceding claims **characterized in that** said article is a diaper or training pant, said article having an uneven thickness across its surface and being folded and rolled such that said thickness difference does not result in a pressure difference in said packaged article of more than 25%, preferably not more than 15%, most preferably not more than 10% between any two points along the length of said packaged article, based on the lower of any two pressures measured.

9. A packaged absorbent article according to any preceding claims **characterized in that** said package further comprises an additional item selected from the group of information or advertisement leaflets, disposal bags, change mats, a dry or wet wipe, toys or gimmicks, a second disposable absorbent article, preferably of identical design as the first disposable article, or combinations thereof.
